# EUROPÄISCHE PATENTSCHRIFT

(11) **EP 2 091 701 B1**
(45) Veröffentlichungstag und Bekanntmachung des Hinweises auf die Patenterteilung: **14.08.2013**
(21) Anmeldenummer: 07818573.3
(22) Anmeldetag: 29.09.2007
(51) Int. Cl.: B26B 19/38, A45D 26/00

(54) **WASCHBARES GERÄT DES PERSÖNLICHEN BEDARFS, INSBESONDERE HAARENTFERNUNGSGERÄT**
WASHABLE PERSONAL GROOMING DEVICE, IN PARTICULAR HAIR REMOVAL DEVICE
APPAREIL LAVABLE À USAGE PERSONNEL, EN PARTICULIER APPAREIL D'ÉPILATION

(30) Priorität: 16.11.2006 DE 102006054027
(43) Veröffentlichungstag der Anmeldung: 26.08.2009
(73) Patentinhaber: Braun GmbH, 61476 Kronberg/Taunus (DE)
(72) Erfinder: DAMASCHKE, Robert, 65760 Eschborn (DE)
(86) Internationale Anmeldenummer: PCT/EP2007/008493
(87) Internationale Veröffentlichungsnummer: WO 2008/058594

(56) Entgegenhaltungen:
- EP-A- 0 478 246
- AT-U1- 6 261
- AT-U1- 6 461
- DE-U1-202006 005 671
- JP-A- 2006 223 475
- KR-A- 20040 096 930
- US-A- 2 022 457
- US-A- 2 281 434
- US-A- 5 294 251

## Beschreibung

Die Erfindung betrifft ein waschbares Gerät des persönlichen Bedarfs, insbesondere Haarentfernungsgerät, wie beispielsweise Epilierer und Rasieren.

### Stand der Technik

Geräte der hier angesprochenen Art sind hinlänglich bekannt. So beschreibt beispielsweise die DE 10 2004 047 874 A1 ein Haarentfernungsgerät in Art eines Epilierers. Ferner ist aus der DE 41 28 217 A1 ein Rasierer bekannt. All diese Geräte kommen immer wieder mit Wasser und anderen Flüssigkeiten in Kontakt. Sie werden mit Flüssigkeit gereinigt bzw. abgewaschen. Darüber hinaus kommen beispielsweise Nassrasierer auch während ihrer Benutzung in Kontakt mit Flüssigkeiten. Die Flüssigkeit dringt in Öffnungen und kleinste Hohlräume der Geräte, insbesondere in innen liegende Gehäusebereiche, ein und verbleibt dort. Dabei ist ein schnelles Abtrocknen der Flüssigkeit in diesen Bereichen üblicherweise nicht gegeben.

Die österreichische Gebrauchsmusterschrift AT 006 461 U1 offenbart einen Rahmen für einen Scher- oder Epilationskopf eines elektrischen Rasier- bzw. Epilationsgerätes. Dieser Rahmen kann mit einer Lackierung versehen werden, welche vor Zerkratzungen geschützt werden soll. Der Rahmen kann daher mit einer Polytetrafluorethylenbeschichtung versehen werden.

US 5,924,251 offenbart eine chemische Zusammensetzung, welche mikrokristallines Paraffinwachs und ein organisches Lösungsmittel enthält. Diese chemische Zusammensetzung dient beispielsweise dazu, Textilien wasserabweisend zu machen oder als Korrosionsschutz für Metalle.

Die österreichische Gebrauchsmusterschrift AT 006 261 U1 offenbart einen elektrischen Rasierapparat, welcher sowohl eine Nass- als auch eine Trockenreinigung ermöglichen soll, indem an dem elektrischen Rasierapparat bestimmte Rasteinrichtungen vorgesehen sind.

Das US-Patent 2,022,457 offenbart eine elektrisch antreibbare Zahnbürste, bei der die Antriebseinheit in Wachs eingebettet werden kann.

Die Gebrauchsmusterschrift DE 202006005671 U1 offenbart eine Emulsion aus Wachs und Wasser, welche auf Verbindungselemente, beispielsweise Schrauben oder Muttern, aufgetragen werden kann und dort die Reibwerte beim Verschrauben beeinflußt und als Korrosionsschutz dient.

Jene Einlagerung von Flüssigkeit in den Geräten ist vor allem vor dem Hintergrund problematisch, dass bei der Benutzung der Geräte, beispielsweise beim Rasieren oder dem Epilieren, Haut- und/oder Haarpartikel freigesetzt werden, welche in der Flüssigkeit zum Gerät gelangen und sich dann in den Geräten einlagern. Aufgrund der im Wohnraum und Badezimmer üblicherweise herrschenden Temperaturen und der relativ langen Verweilzeit der Flüssigkeit in den Geräten können sich sehr schnell unerwünschte Krankheitserreger in der Art von Keimen oder Bakterien bilden und vermehren. Mit der Zeit entsteht dadurch eine erhebliche Infektionsgefahr für den Benutzer des Gerätes.

### Aufgabenstellung

Davon ausgehend liegt der Erfindung die Aufgabe zugrunde, ein waschbares Gerät des persönlichen Bedarfs mit den eingangs genannten Merkmalen vorzuschlagen, bei welchem eine Bildung und Vermehrung von gefährlichen Krankheitserregern vermieden wird. Ferner soll ein Verfahren zum Herstellen von Bauteilen eines solchen Gerätes des persönlichen Bedarfs vorgeschlagen werden.

### Erfindung und vorteilhafte Wirkungen

Zur Lösung der Aufgabe wird ein waschbares Gerät des persönlichen Bedarfs, insbesondere Haarentfernungsgerät, vorgeschlagen, welches die in Anspruch 1 genannten Merkmale aufweist. Das Gerät zeichnet sich unter anderem dadurch aus, dass es zumindest teilweise mit Paraffin beschichtet ist.

Bei der erfindungsgemäßen Maßnahme macht man sich die hydrophobe Eigenschaft von Paraffin zu Nutze. Diese bewirkt, dass auf den beschichteten Oberflächen die auftreffende Flüssigkeit abperlt. Insofern kann bereits durch leichte Schüttelbewegungen des Gerätes ein Abfließen der Flüssigkeit, beispielsweise aus Öffnungen und kleinsten Hohlräumen, erreicht werden. Die tribologischen Eigenschaften von derart behandelten Oberflächen (mit z. B. Paraffin) werden somit positiv verändert.

Dadurch kann die Verweilzeit der Flüssigkeit in den Geräten soweit verringert werden, dass Krankheitserreger erst gar nicht entstehen können oder zumindest eine Vermehrung verhindert ist. Gesundheitliche Risiken durch die Verwendung der Geräte werden damit vermieden.

Nach einer ersten Ausgestaltung der Erfindung ist es vorgesehen, dass Oberflächen einzelner Bauteile des Gerätes mit Paraffin beschichtet sind. Hierdurch kann mit geringstem Aufwand und somit relativ kostengünstig der Bildung und Vermehrung von Krankheitserregern vorgebeugt werden, indem gezielt nur die Oberflächen derjenigen Bauteile beschichtet werden, in welchen es zur Einlagerung von Flüssigkeit kommt und durch welche ein Abfließen der Flüssigkeit bisher erschwert ist.

Dabei bietet es sich an, dass die beschichteten Oberflächen der Bauteile innerhalb des Gehäuses des Gerätes liegen. Hierdurch ist gezielt eine Beschichtung derjenigen Bauteile vorgesehen, in welchen sich die Flüssigkeit bevorzugt einlagert, sodass dadurch besonders wirkungsvoll der Bildung und Vermehrung von Krankheitserregern entgegengewirkt wird.

Zusätzlich oder alternativ kann es vorgesehen sein, dass die beschichteten Oberflächen der Bauteile außerhalb des Gehäuses des Gerätes liegen. Hierdurch ist das Abfließen der Flüssigkeit auch über die außerhalb des Gehäuses liegenden Bauteile begünstigt.

In die gleiche Richtung geht die Maßnahme, dass im Innenraum liegende Oberflächen des Gehäuses beschichtet sind.

Nach einer weiteren Ausgestaltung der Erfindung ist es vorgesehen, dass die Paraffinschicht aus Hartparaffin besteht. Jene Hartparaffine sind aufgrund ihrer Geruchsneutralität und ihrer Ungiftigkeit, chemischen Beständigkeit und des großen Bereichs der Schmelzpunkteinstellung besonders für den Einsatz in waschbaren Geräten des persönlichen Bedarfs geeignet.

Nach einer weiteren Ausgestaltung der Erfindung sind Mittel zum Abführen von Flüssigkeit vorgesehen. Durch diese Maßnahme wird das Abfließen der Flüssigkeit, insbesondere der aufgrund der Paraffinbeschichtung vorliegenden tropfenförmigen Flüssigkeit, begünstigt.

Dabei bietet es sich an, dass die Mittel durch Bohrungen, Schlitze, Nuten und/oder dergleichen Ablaufeinrichtungen am und/oder im Gerät gebildet sind. Hierdurch kann gezielt je nach Art der Einlagerung der Flüssigkeit, beispielsweise in kleinsten Hohlräumen oder innerhalb des Gehäuses des Gerätes eine entsprechende Ablaufeinrichtung realisiert und somit die Flüssigkeit wirkungsvoll abgeführt werden.

Nach einer weiteren Ausführungsform der Erfindung ist es vorgesehen, dass das Gehäuse und/oder die Bauteile zumindest im beschichteten Bereich aus Kunststoff bestehen. Hierdurch ist trotz der Paraffinbeschichtung des Gerätes dessen Herstellung kostengünstig möglich. Besonders kostengünstig ist es, wenn die Bauteile vollständig aus Kunststoff gebildet sind.

Nach einer weiteren Ausgestaltung der Erfindung ist es vorgesehen, dass die Paraffinschicht einen antibakteriell wirkenden Stoff enthält. Durch diese Maßnahme kommt der auf die Bauteile bzw. auf das Gehäuse aufgetragenen Schicht neben einer das Abfließen der Flüssigkeit begünstigenden Funktion zusätzlich eine die Bildung und Vermehrung von Krankheitserregern hemmende Funktion zu. Damit ist in optimaler Art und Weise der Bildung und Vermehrung gesundheitsgefährdender Krankheitserreger, wie Bakterien, Keime und dergleichen, bei den Geräten vorgebeugt.

Nach einer Weiterbildung der Erfindung ist es ferner vorgesehen, dass das Gerät antibakteriell wirkenden Stoff enthält. Damit sind neben der Beschichtung auch das Gerät bzw. dessen Bauteile mit antibakteriell wirkendem Stoff versehen. Durch diese Maßnahme ist gewährleistet, dass auch Bereiche ohne Beschichtung eine antibakterielle Wirkung entfalten, sodass dort ebenfalls die Bildung wie auch die Vermehrung von Krankheitserregern gehemmt ist. Dieser Aspekt kann auch als eigenständiger Gedanke der Erfindung gesehen werden.

Bevorzugt ist der antibakteriell wirkende Stoff zumindest in oberflächennahen Schichten des Gehäuses des Gerätes und/oder einzelner Bauteile des Gerätes im Innen- und/oder Außenraum des Gerätes angeordnet. Dadurch ist eine optimale Entfaltung der antibakteriellen Wirkung gewährleistet, da gerade an bzw. in den oberflächennahen Schichten die Bildung und Vermehrung der Krankheitserreger stattfindet.

Mit Vorteil ist es ferner vorgesehen, dass der antibakteriell wirkende Stoff in Bauteilen enthalten ist, welche bei der Benutzung des Gerätes in direkten Kontakt mit Haut kommen. Dadurch wird zum einen einer Übertragung von möglicherweise bereits im Gerät gebildeten Krankheitserregern auf die Haut entgegengewirkt.

Um trotzdem das Gerät besonders kostengünstig herstellen zu können, bietet es sich an, dass das Gehäuse und/oder die Bauteile zumindest in den oberflächennahen Schichten aus Kunststoff bestehen. Hierdurch kann insbesondere der antibakteriell wirkende Stoff bei der Herstellung der Bauteile einfach eingegossen werden, sodass die Herstellung von Bauteilen mit antibakteriell wirkendem Stoff kostengünstig realisierbar ist.

Von Vorteil ist es dabei, dass der antibakteriell wirkende Stoff im Wesentlichen gleichmäßig verteilt vorliegt, um eine entsprechend gleichmäßige Wirkung über die vorgesehene Oberfläche der Bauteile zu gewährleisten.

Mit Vorteil ist als antibakteriell wirkender Stoff Silber vorgesehen. Silber ist beispielsweise auf unterschiedlichen Trägermaterialien als Batch für Kunststoffe bereits als Massenprodukt erhältlich. Insofern ist dieser antibakteriell wirkende Stoff in den Geräten kostengünstig zu realisieren. Darüber hinaus wird dem Silber eine besonders wirkungsvolle antibakterielle Wirkung nachgesagt. Diese Wirkung beruht auf freigesetzten Silber-Ionen, welche ab einer gewissen Konzentration eine antibakterielle und eine fungizide Wirkung hervorrufen.

Bevorzugt sollte der Anteil des Silbers im Gehäuse und/oder in den Bauteilen und/oder in der Paraffinschicht größer als 5 ppm oder bevorzugt größer 20 ppm oder zwischen 10 und 130 ppm liegen. Eine besonders gute Wirkung des Silbers kann erreicht werden, wenn der Anteil des Silbers zwischen 10 und 100 ppm liegt. Bevorzugt sollte der Anteil des Silbers zwischen 15 und 60 ppm liegen, da in diesem Konzentrationsbereich eine optimale antibakterielle und fungizide Wirkung erzielbar ist.

Nach einer Weiterbildung der Erfindung ist es vorgesehen, dass bei aneinander gleitenden Flächen des Gerätes wenigstens eine der Flächen paraffinbeschichtet ist. Insofern wird sich die Eigenschaft des Paraffins als Schmiermittel zu Nutze gemacht. Durch das Paraffin ist eine Schmierung über die gesamte Lebensdauer des Gerätes ermöglicht. Aufwendiges Nachschmieren ist nicht notwendig. Dies beruht im Wesentlichen auf den vorteilhaften Eigenschaften des Paraffins, wonach es beständig gegen die meisten haushaltsüblichen Kosmetik-/Reinigungschemikalien und beständig gegen eine mechanische Reinigung, wie beispielsweise mittels Bürsten, sowie in einem weiten Temperaturbereich einsetzbar ist. Beim Paraffin kommt es daher zu keinem Auswaschen durch Tenside, wie Seifen, Geschirrspülmittel, Alkohole und dergleichen, wie das bei den bisher eingesetzten Schmierfetten der Fall ist.

Mit Vorteil ist es vorgesehen, dass die beschichteten Oberflächen eine raue, vorzugsweise eine Schmiermittelreservoir bildende Struktur, aufweisen. Hierdurch kann selbst bei einem gewissen Verschleiß der Paraffinschicht eine ausreichende Schmierung sichergestellt werden, indem die Oberflächenstruktur Einschlüsse aufweist, welche als Paraffinreservoir dienen.

Mit Vorteil ist es auch vorgesehen, dass die aneinander gleitenden Flächen eine Materialpaarung aus Metall, Kunststoff und/oder Keramik bilden. Bei diesen Materialpaarungen kann mittels der Paraffinbeschichtung eine besonders gute Lebensdauerschmierung erzielt werden. Dadurch ist beispielsweise eine Schmierung in Lagern, Führungen, Antrieben und dergleichen Bewegungsübertragungsgliedern problemlos möglich.

Zur Lösung der Aufgabe ist ferner ein Verfahren zum Herstellen von Bauteilen eines Gerätes des persönlichen Bedarfs, insbesondere Haarentfernungsgerät, wie beispielsweise Epilierer und Rasierer, vorgesehen. Das Verfahren zeichnet sich unter anderem dadurch aus, dass das Bauteil in eine Schmelze aus Paraffin eingetaucht und/oder mit der Schmelze besprüht wird, oder dass das Bauteil mit einer Paraffinlösung besprüht oder mit festem Paraffin getrommelt wird.

Durch diese Maßnahme ist eine besonders feste und damit haltbare Paraffinbeschichtung auf den Bauteilen für das Gerät, wie beispielsweise einem Haarentfernungsgerät in Art eines Epilierers oder Rasierers, realisierbar.

### Ausführungsbeispiele

Weitere Ziele, Vorteile, Merkmale und Anwendungsmöglichkeiten der vorliegenden Erfindung ergeben sich aus der nachfolgenden Beschreibung von Ausführungsbeispielen anhand der Zeichnungen.
- Figur 1: ein mögliches Ausführungsbeispiel eines waschbaren Gerätes in Art eines Epilierers in perspektivischer Darstellung des oberen Teiles der Gerätes,
- Figur 2: einen Teil des Walzenpaketes des Gerätes gemäß Figur 1 in einer ersten Betriebsstellung als perspektivische Darstellung,
- Figur 3: den Teil des Walzenpaketes gemäß Figur 2 in einer zweiten Betriebsstellung als perspektivische Darstellung,
- Figur 4: das obere Teil des Gerätes gemäß Figur 1 in perspektivischer Ansicht von unten,
- Figur 5: eine Andruckplatte für ein Oberteil eines bekannten Epilierers mit umlaufenden Rillen in perspektivischer Ansicht,
- Figur 6: eine Andruckplatte des Epilierers gemäß Figur 1 in perspektivischer Ansicht,
- Figur 7: ein weiteres mögliches Ausführungsbeispiel eines Haarentfernungsgerätes in Art eines Rasierers als Teildarstellung in perspektivischer Ansicht und

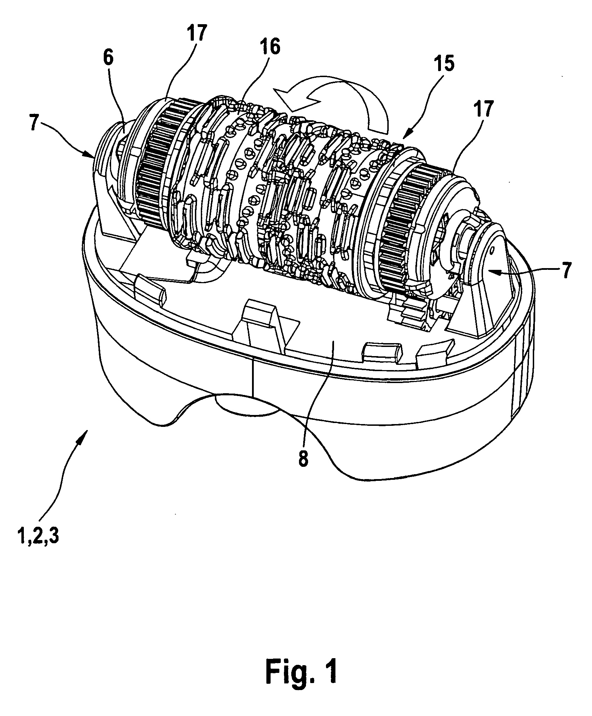

Figuren 1 bis 3 zeigen einen Teil eines waschbaren Gerätes 1 des persönlichen Bedarfs in Art eines Haarentfernungsgerätes 2. Das dort dargestellte Haarentfernungsgerät 2 ist ein Epilierer 3, wobei lediglich dessen oberer Teil bzw. Kopfteil dargestellt ist.

Das Kopfteil des Epilierers 3 weist ein Walzenpaket 15 mit einer Epilationswalze 16 und darin befindlichen Bauteilen 5 in Art von Stößeln auf. Die Epilationswalze 16 ist zwischen zwei Andruckplatten 17 gehalten, wobei die Andruckplatten 17 eine Betätigungskraft an die Stößel 5 weiterleiten. Durch die Rotation der Epilationswalze 16 gleiten die Stößel 5 mit einem Ende in einer jeweils an der Innenseite der Andruckplatten 17 angeordneten Führung in einer vorgegebenen Bahnkurve. Insoweit sind die beschriebenen Merkmale an sich bereits bekannt.

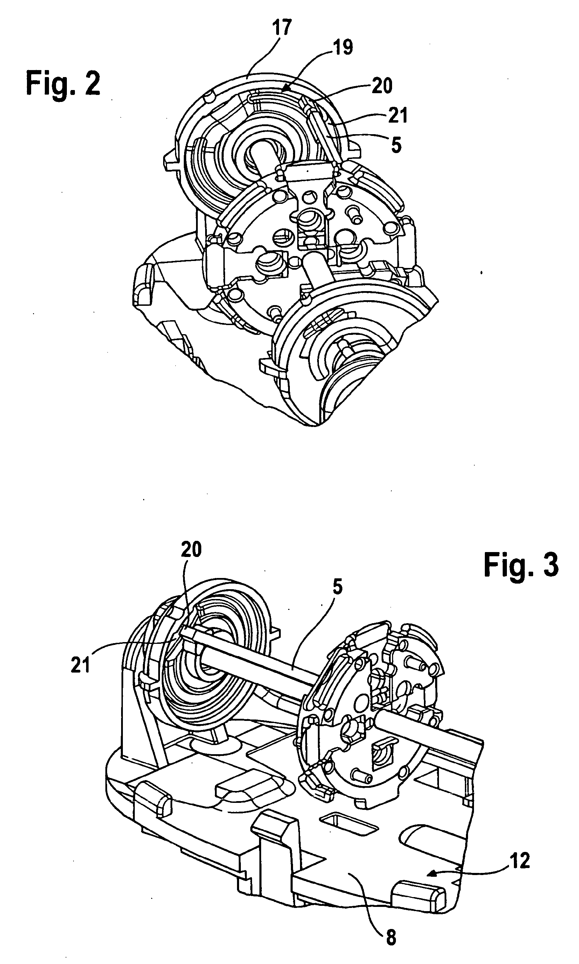

Figur 2 zeigt exemplarisch einen der Stößel 5 in einer ersten Lage an einer der Andruckplatten 17 und Figur 3 diesen Stößel 5 in einer zweiten Lage, in der der Stößel 5 entlang der Bahnkurve bereits eine gewisse Bewegung vollzogen hat.

Im Unterschied zu den bekannten Epilierern ist bei dem Epilierer gemäß der Figuren 1 bis 3 eine Beschichtung mit Paraffin, vorzugsweise Hartparaffin, zumindest eines Teils des Epilierers 3 vorgesehen. Dabei sind Oberflächen einzelner Bauteile mit Paraffin beschichtet.

Bei dem Ausführungsbeispiel gemäß der Figuren 1 bis 3 sind die Oberflächen der Bauteile 7 und 8 mit Paraffin beschichtet. Bei dem Bauteil 7 handelt es sich um die seitlichen Lager für die Epilationswalze 16, in welche sich insbesondere beim Abwaschen des Epilierers 3 bevorzugt Flüssigkeit in den vorhandenen Hohlräumen anlagert. Bei dem Bauteil 8 handelt es sich um das Bodenteil des Kopfteiles des Epilierers 3. Jenes Bodenteil 8 bildet mit einer in den Figuren 1 bis 3 nicht dargestellten Abdeckung für die Epilationswalze 16 ein das Kopfteil des Epilierers 3 umgebendes Gehäuse.

Dieses (nicht vollständig dargestellte) Gehäuse kann an den der Epilationswalze 16 zugewandten Oberfläche mit Paraffin beschichtet sein.

Durch die Beschichtung der Bauteile des Epilierers 3, wie sie beispielsweise bei dem Ausführungsbeispiel gemäß der Figuren 1 bis 3 an den Bauteilen 7 und 8 vorgenommen ist, kommt es zu einem Abperlen der auf diese Oberflächen auftreffenden Flüssigkeit. Dadurch ist ein Abführen dieser perlenden Flüssigkeit aus dem Epilierer 3 begünstigt.

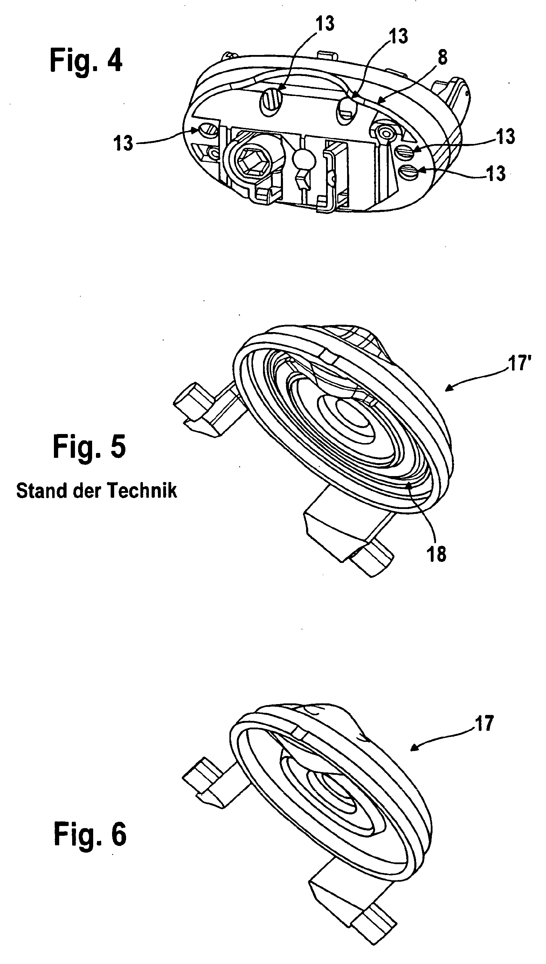

Weiterhin sind entsprechende Mittel 13 zum Abführen vorgesehen, wie aus Figur 4 ersichtlich ist. Die Mittel 13 zum Abführen von Flüssigkeit sind dort als Bohrungen bzw. Öffnungen im Boden 8 des Kopfteils des Epilierers 3 vorgesehen.

Eine Paraffinbeschichtung ist bei dem Ausführungsbeispiel gemäß der Figuren 1 bis 3 auch bei aneinander gleitenden Flächen vorgesehen. Solche Flächen sind beispielsweise die Flächen 21 der Führung 19 der Andruckplatten 17, welche in Gleitkontakt mit den Flächen 20 der Enden der Stößel 5 stehen. Dabei kann neben dem Ende der Stößel 5 zusätzlich auch die Führung 19 der Andruckplatten 17 oder die gesamten Andruckplatten 17 mit Paraffin beschichtet sein. Auch ist zwischen den Enden der Welle 6 der Epilationswalze 16 und den seitlichen Lagern 7 die Gleitfläche mit Paraffin beschichtet. Insofern ist durch die Beschichtung eine Schmierung gewährleistet. Wie die beschichteten Bauteile 5 und 6 und gegebenenfalls 17 gemäß dem Ausführungsbeispiel der Figuren 1 bis 3, sind vorzugsweise solche Bauteile beschichtet, welche innerhalb des (nicht dargestellten) Gehäuses des Epilierers 3 liegen.

Bevorzugt weisen bei den aneinander gleitenden Flächen 20, 21 von Stößel 5 und Führung 19 wie auch bei sämtlichen anderen vorgesehenen aneinander gleitenden Flächen des Epilierers 3 die beschichteten Oberflächen eine raue, vorzugsweise eine ein Schmiermittelreservoir bildende Struktur auf. Bei dem Ausführungsbeispiel gemäß der Figuren 1 bis 3 weist die Oberfläche der aneinander gleitenden Teile bevorzugt eine Oberflächenrauigkeit in einem Bereich zwischen 10 bis 30 µm auf.

Bei der Ausführungsform gemäß der Figuren 1 bis 3 sind sämtliche Paraffinbeschichtungen mit einem Anteil von 20 bis 50 ppm Silber versehen. Damit ist zum einen mittels der Paraffinbeschichtung die Möglichkeit gegeben, dass auf die Oberflächen treffende Flüssigkeit in Tropfen abperlen und somit ein Abführen der Flüssigkeit von dem Epilierer 3 begünstigt ist. Daneben ist durch den zusätzlich in Paraffin enthaltenen Silberanteil eine antibakterielle Wirkung erzielt, sodass durch die Paraffinschicht und den Silberanteil eine die Entstehung von gefährlichen Keimen und deren Vermehrung vermieden wird.

Selbstverständlich kann auch das Material der Bauteile selbst einen antibakteriell wirkenden Stoff, wie beispielsweise Silber, enthalten. Bevorzugt ist der Anteil dieses Stoffes im Material ebenfalls 20 bis 50 ppm.

Die beschichteten Bauteile 5, 6, 7, 8 sind vorliegend aus Kunststoff hergestellt. Aufgrund der hohen Reibungsminderung durch die Paraffinbeschichtung bei den in Gleitkontakt stehenden Bauteilen 6, 7 bzw. 5, 17, ist es jedoch auch möglich, dass das Material der aneinander gleitenden Flächen aus Kunststoff besteht.

Wie aus den Figuren 5 und 6 ersichtlich ist, ist mittels der Paraffinbeschichtung konstruktiv eine Vereinfachung der seitlichen Andruckplatten 17 erreicht. In Figur 5 ist ein Beispiel einer bisher bekannten Andruckplatte 17' dargestellt. Dort befinden sich umlaufende Rillen 18 in der Andruckplatte, welche als Schmiermittelreservoire für die bei den bekannten Andruckplatten 17' verwendeten Schmierfette eingesetzt werden. Derartige umlaufende Rillen sind bei den Andruckplatten 17 des Epilierers 3 gemäß der Figuren 1 bis 3 nicht mehr erforderlich.

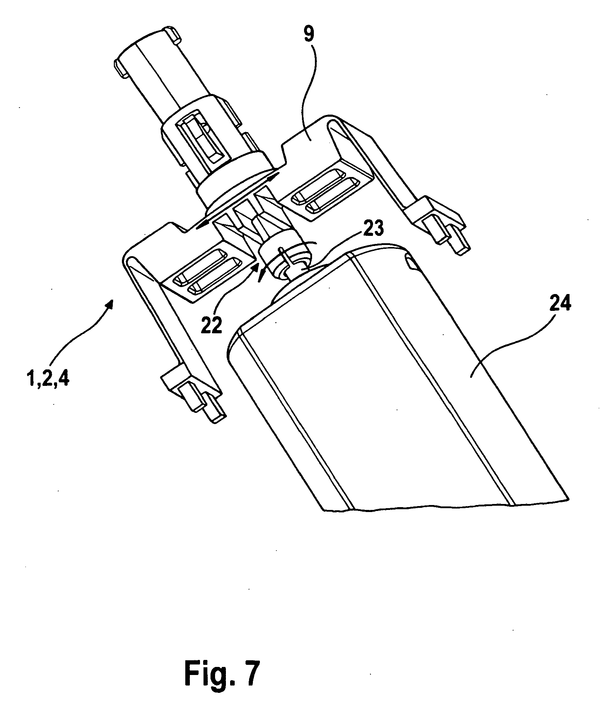

Figur 7 zeigt ein weiteres Ausführungsbeispiel des waschbaren Gerätes 1 des persönlichen Bedarfs in Art des Haarentfernungsgerätes 2. Das dort dargestellte Haarentfernungsgerät 2 ist ein elektrischer Rasierer 4 mit einer Schwingbrücke 9, welche eine Exzenter-Einrichtung 22 aufweist. Diese Exzenter-Einrichtung wird von einer rotierenden Abtriebswelle 23 eines Elektromotors 24 in eine translatorische Bewegung versetzt.

Bei dem Ausführungsbeispiel gemäß Figur 7 sind die in der Exzenter-Einrichtung 22 gegeneinander gleitenden Flächen mit Paraffin beschichtet, um dadurch eine Lebensdauerschmierung zu erreichen.

Darüber hinaus ist als eines der Bauteile die Schwingbrücke 9 vorzugsweise vollständig mit Paraffin beschichtet, um dadurch ein Abperlen der auf die Schwingbrücke auftreffenden Flüssigkeit zu erzielen.

Selbstverständlich können auch die aneinander gleitenden Flächen eines auf den Rasierer 4 aufsteckbaren (nicht dargestellten) Langhaarschneiders mit Paraffin beschichtet sein. In gleicher Weise können sämtliche Bauteile des Langhaarschneiders als solches eine Paraffinbeschichtung aufweisen.

## Patentansprüche

1. Waschbares Gerät (1) des persönlichen Bedarfs, insbesondere Haarentfernungsgerät (2), wie beispielsweise Epilierer (3) und Rasierer (4), wobei Oberflächen einzelner Bauteile (5, 6, 7, 8; 9) des Gerätes (1, 2, 3, 4) mit Paraffin beschichtet sind und die beschichteten Oberflächen der Bauteile (5, 6, 7, 8; 9) innerhalb des Gehäuses des Gerätes (1, 2, 3, 4) liegen, **dadurch gekennzeichnet, dass** die Bauteile (5, 6, 7, 8; 9) zumindest im beschichteten Bereich aus Kunststoff bestehen.

2. Gerät nach Anspruch 1, **dadurch gekennzeichnet, dass** die Paraffinschicht aus Hartparaffin besteht.

3. Gerät nach einem der vorhergehenden Ansprüche, **dadurch gekennzeichnet, dass** Mittel (13) zum Abführen von Flüssigkeit vorgesehen sind.

4. Gerät nach Anspruch 3, **dadurch gekennzeichnet, dass** die Mittel durch Bohrungen (13), Schlitze, Nuten und/oder dergleichen Ablaufeinrichtungen am und/oder im Gerät (1, 2, 3) gebildet sind.

5. Gerät nach einem der vorhergehenden Ansprüche, **dadurch gekennzeichnet, dass** die Paraffinschicht einen antibakteriell wirkenden Stoff enthält.

6. Gerät nach einem der vorhergehenden Ansprüche, **dadurch gekennzeichnet, dass** das Gerät (1, 2, 3, 4) antibakteriell wirkenden Stoff enthält.

7. Gerät nach Anspruch 5 oder 6, **dadurch gekennzeichnet, dass** der antibakteriell wirkende Stoff zumindest in oberflächennahen Schichten des Gehäuses des Gerätes (1, 2, 3, 4) und/oder einzelner Bauteile (5, 6, 7, 8; 9)des Gerätes (1, 2, 3, 4) im Innen- und/oder Außenraum des Gehäuses angeordnet ist.

8. Gerät nach einem der Ansprüche 5 bis 7, **dadurch gekennzeichnet, dass** der antibakteriell wirkende Stoff in Bauteilen enthalten ist, welche bei der Benutzung des Gerätes in direkten Kontakt mit der Haut kommen.

9. Gerät nach einem der Ansprüche 7 oder 8, **dadurch gekennzeichnet, dass** das Gehäuse und/oder die Bauteile (5, 6, 7, 8; 9) zumindest in den oberflächennahen Schichten aus Kunststoff bestehen.

10. Gerät nach einem der Ansprüche 5 bis 9, **dadurch gekennzeichnet, dass** Silber als antibakteriell wirkender Stoff vorgesehen ist.

11. Gerät nach Anspruch 10, **dadurch gekennzeichnet, dass** der Anteil des Silbers im Gehäuse und/oder in den Bauteilen (5, 6, 7, 8; 9) und/oder in der Paraffinschicht größer 5 ppm, bevorzugt größer 20 ppm oder zwischen 10 und 130 ppm, vorzugsweise zwischen 10 und 100 ppm, vorzugsweise zwischen 15 und 60 ppm, liegt.

12. Gerät nach einem der vorhergehenden Ansprüche, **dadurch gekennzeichnet, dass** bei aneinander gleitenden Flächen (20, 21) des Gerätes (1, 2, 3) wenigstens eine der Flächen (20; 21) mit Paraffin beschichtet ist.

13. Gerät nach Anspruch 12, **dadurch gekennzeichnet, dass** die beschichteten Oberflächen eine raue, vorzugsweise eine Schmiermittelreservoir bildende Struktur, aufweisen.

14. Gerät nach Anspruch 12 oder 13, **dadurch gekennzeichnet, dass** die aneinander gleitenden Flächen (20, 21) eine Materialpaarung aus Metall, Kunststoff und/oder Keramik bilden.

## Claims

1. A washable apparatus (1) for personal use, particularly a hair removal apparatus (2), e.g. an epilator (3) and a shaver (4), wherein the surfaces of individual component parts (5, 6, 7, 8; 9) of the apparatus (1, 2, 3, 4) are coated with paraffin, and the coated surfaces of the component parts (5, 6, 7, 8; 9) are disposed inside the housing of the apparatus (1, 2, 3, 4), **characterized in that the** component parts (5, 6, 7, 8; 9) are comprised of plastic, at least in the coated region.

2. An apparatus according to claim 1, **characterized in that the** paraffin coating is comprised of hard paraffin.

3. An apparatus according to one of the preceding claims, **characterized in that means** (13) for removal of liquid are provided.

4. An apparatus according to claim 3, **characterized in that the** means (13) for removal of liquid comprise openings, bores, slots, grooves, and/or similar removal means formed on or in the apparatus (1, 2, 3).

5. An apparatus according to one of the preceding claims, **characterized in that the** paraffin coating has an anti-bacterially active material.

6. An apparatus according to one of the preceding claims, **characterized in that the** apparatus (1, 2, 3, 4) contains anti-bacterially active material.

7. An apparatus according to claim 5 or 6, **characterized in that the** anti-bacterially active material is disposed at least in layers of the housing of the apparatus (1, 2, 3, 4) which are near the surface, and/or in individual component parts (5, 6, 7, 8; 9) of the apparatus (1, 2, 3, 4) in the interior and/or outer space of the housing.

8. An apparatus according to one of claims 5-7, **characterized in that the** anti-bacterially active material is contained in component parts, which come into direct contact with the skin when the apparatus is used.

9. An apparatus according to one of claim 7 or 8, **characterized in that the** housing and/or the component parts (5, 6, 7, 8; 9) are comprised of plastic, at least in layers near the surface.

10. An apparatus according to one of claims 5-9, **characterized in that silver** is provided as an anti-bacterially active material.

11. An apparatus according to claim 10, **characterized in that the** proportion of silver in the housing and/or in the components (5, 6, 7, 8; 9) and/or in the paraffin coating is greater than 5 ppm, preferably greater than 20 ppm, or between 10 and 130 ppm, preferably between 10 and 100 ppm, particularly preferably between 15 and 60 ppm.

12. An apparatus according to one of the preceding claims, **characterized in that, where** the apparatus (1, 2, 3) has mutually gliding surfaces (20, 21), at least one of the surfaces (20; 21) is coated with paraffin.

13. An apparatus according to claim 12, **characterized in that the** coated surfaces have a rough structure, preferably of a type which forms a lubricant reservoir.

14. An apparatus according to claim 12 or 13, **characterized in that the** mutually gliding surfaces (20, 21) form a material pairing comprised of metal, plastic, and/or ceramic.

## Revendications

1. Appareil lavable (1) à usage personnel, en particulier appareil d'épilation (2), comme par exemple un épilateur (3) et un rasoir (4), dans lequel les surfaces de composants individuels (5, 6, 7, 8 ; 9) de l'appareil (1, 2, 3, 4) sont revêtues de paraffine et les surfaces revêtues des composants (5, 6, 7, 8 ; 9) se trouvent à l'intérieur du boîtier de l'appareil (1, 2, 3, 4), **caractérisé en ce que** les composants (5, 6, 7, 8 ; 9) sont composés au moins dans la zone revêtue de matières plastiques.

2. Appareil selon la revendication 1, **caractérisé en ce que** la couche de paraffine est composée de paraffine dure.

3. Appareil selon une des revendications précédentes, **caractérisé en ce que** des moyens (13) pour évacuer du liquide sont prévus.

4. Appareil selon la revendication 3, **caractérisé en ce que** les moyens sont formés par le biais d'alésages (13), de fentes, de rainures et/ou de dispositifs de vidange similaires sur et/ou dans l'appareil (1, 2, 3).

5. Appareil selon une des revendications précédentes, **caractérisé en ce que** la couche de paraffine contient un matériau à effet antibactérien.

6. Appareil selon une des revendications précédentes, **caractérisé en ce que** l'appareil (1, 2, 3, 4) contient un matériau à effet antibactérien.

7. Appareil selon la revendication 5 ou 6, **caractérisé en ce que** le matériau à effet antibactérien est placé au moins dans les couches proches de la surface du boîtier de l'appareil (1, 2, 3, 4) et/ou des composants individuels (5, 6, 7, 8 ; 9) de l'appareil (1, 2, 3, 4) dans l'espace interne et/ou externe du boîtier.

8. Appareil selon une des revendications 5 à 7, **caractérisé en ce que** le matériau à effet antibactérien est contenu dans les composants, qui viennent en contact direct avec la peau lors de l'utilisation de l'appareil.

9. Appareil selon une des revendications 7 ou 8, **caractérisé en ce que** le boîtier et/ou les composants (5, 6, 7, 8 ; 9) sont composés de matières plastiques, au moins dans les couches proches de la surface.

10. Appareil selon une des revendications 5 à 9, **caractérisé en ce que** fagent à effet antibactérien est l'argent.

11. Appareil selon la revendication 10, **caractérisé en ce que** la part d'argent dans le boîtier et/ou dans les composants (5, 6, 7, 8 ; 9) et/ou dans la couche de paraffine est supérieure à 5 ppm, de préférence supérieure à 20 ppm ou comprise entre 10 et 130 ppm, de manière préférée comprise entre 10 et 100 ppm, de préférence entre 15 et 60 ppm.

12. Appareil selon une des revendications précédentes, **caractérisé en ce que,** parmi les surfaces glissant l'une contre l'autre (20, 21) de l'appareil (1, 2, 3) au moins une des surfaces (20 ; 21) est revêtue de paraffine.

13. Appareil selon la revendication 12, **caractérisé en ce que** les surfaces revêtues présentent une structure rugueuse, formant de préférence un réservoir de lubrifiant.

14. Appareil selon la revendication 12 ou 13, **caractérisé en ce que** les surfaces glissant l'une contre l'autre (20, 21) forment une combinaison de matériau métal, matières plastiques et/ou céramique.
